# Europäisches Patentamt

# European Patent Office

# Office européen des brevets

(11) Veröffentlichungsnummer: **0 135 208**
**B1**

---

(12) **EUROPÄISCHE PATENTSCHRIFT**

---

(45) Veröffentlichungstag der Patentschrift:
22.03.89

(51) Int. Cl.⁴: **C 12 M 1/12,** C 12 M 1/26,
C 12 N 1/02

(21) Anmeldenummer: 84201005.0

(22) Anmeldetag: 10.07.84

(54) Verfahren zur Abtrennung von Hefen aus Fermentationsbrühen sowie dessen Anwendung.

---

(30) Priorität: 27.07.83 CH 4106/83

(43) Veröffentlichungstag der Anmeldung:
27.03.85 Patentblatt 85/13

(45) Bekanntmachung des Hinweises auf die Patenterteilung:
22.03.89 Patentblatt 89/12

(84) Benannte Vertragsstaaten:
AT BE CH DE FR GB IT LI LU NL SE

(56) Entgegenhaltungen:
CH-A- 4 259
DE-B- 1 039 976
FR-A- 2 352 055

JOURNAL OF APPLIED CHEMISTRY
BIOTECHNOLOGY, Band 27, 1977, Seiten 99-109; A.
RUSHTON et al.: "The filtration characteristics of
yeast"

(73) Patentinhaber: DrM.Dr. Hans Müller AG, Alte
Landstrasse 421, CH-8708 Männedorf (CH)

(72) Erfinder: Müller, Hans, Dr.-Ing., im Allmendli 11,
CH-8703 Erlenbach ZH (CH)

---

**Beschreibung**

Die Erfindung betrifft ein Verfahren zur Abtrennung von Hefen aus den Fermentationsbrühen eines Gärtanks, insbesondere zur Herstellung von gepreßter Hefe für Backzwecke sowie gepreßter Hefe, die aus der alkoholischen Vergärung bei der Alkohol-, Wein- und Bierbereitung anfällt, sowie dessen Anwendung.

Es ist bekannt, Hefen aus Fermentationsbrühen in Kammerfilterpressen abzutrennen. Bei diesem Verfahren wird die Hefesuspension so lange durch die Filterpresse gepumpt, bis die Kammern vollständig mit Hefe angefüllt sind. Das Verfahren hat den Nachteil, daß der in den Kammern zusammengepreßte Filterkuchen nur schwierig gewaschen werden kann und zu einer Entfernung sowie zur Reinigung der Filterpressen viel Handarbeit erforderlich ist.

Es ist auch bekannt, Bäckereihefe sowie Hefen, die bei der alkoholischen Gärung aus den Fermentationsbrühen anfallen, zunächst mittels hochtouriger Separatoren auf eine Hefekonzentration von 16–18 Gew.-% Trockensubstanz zu konzentrieren, kühl zu lagern und auf einem Vakuumtrommelfilter unter Verwendung eines Filterhilfsmittels auf 29–32 Gew.-% zu entwässern.

Dieses Verfahren hat den Nachteil, daß zum Auswaschen der Hefeseparation in den Separatoren große Wassermengen erforderlich sind, wobei der Wascheffekt relativ gering ist. Ein weiterer Nachteil sind hohe Investitionskosten für zwei verschiedene Entwässerungsgeräte.

Aufgabe der Erfindung ist es, ein vereinfachtes, umweltschonendes und energiesparendes Verfahren zur wirtschaftlichen Abtrennung von Hefe aus den Fermentationsbrühen eines Gärtanks unter weitgehend sterilen Bedingungen mit einem Trockensubstanzgehalt von wenigstens 29 Gew.-% zu schaffen.

Die Aufgabe wird erfindungsgemäß dadurch gelöst, daß die Fermentationsbrühe durch einen Druckfilter mit frei in einem Filterbehälter aufgehängten Filterelementen gepreßt wird, dessen Filterelemente mit feinem Filtergewebe oder Membranen überzogen sind, der Filterbehälter nach dem Aufbau eines Filterkuchens von der Restflüssigkeit entleert, der Hefe-Filterkuchen mit Wasser gewaschen, mit Druckluft oder einem anderen Druckgas ausgeblasen und durch einen Druckstoß mit Druckgas entgegengesetzt zur Filtrationsrichtung von den Filtergeweben gelöst und aus dem Filterbehälter ausgetragen wird.

Es hat sich sowohl für die Filtration als auch für die Auswaschung der Hefe als besonders vorteilhaft erwiesen, daß die Filterflächen von allen Seiten für die Suspension frei zugänglich sind. Auch nach dem Aufbau des Filterkuchens ist es vorteilhaft, daß der Hefe-Filterkuchen von allen Seiten frei zugänglich ist, wodurch eine von allen Seiten unbehinderte Auswaschung erfolgt.

In einer bevorzugten Ausgestaltung des Verfahrens hat es sich als besonders vorteilhaft erwiesen, den Druckfilter direkt mit dem Gärtank zu verbinden. Dadurch ist es möglich, die Hefe oder einen Teil der Hefe auch während der Gärung unter sterilen Bedingungen abzutrennen. Dadurch wird auch jeder Kontakt mit der Luft vermieden. Die unter sterilen Bedingungen ausgetragene Hefe kann wieder verwendet werden. Durch die direkte Verbindung mit dem Gärtank ist es auch möglich, in einer Filtrationsstufe die gesamte, in einem Gärtank anfallende Hefe auszufiltrieren. Im Falle der Bierfiltration ermöglicht dies die Bierverluste auf nahezu das theoretisch erzielbare Minimum zu reduzieren.

Gemäß Anspruch 3 ist es zweckmäßig, den Filterbehälter unter einem steten Überdruck zu halten, wodurch das Eindringen von Fremdkeimen verhindert wird.

Gemäß Anspruch 4 kann die Waschflüssigkeit in die Ausblasluft oder ein anderes Ausblasgas dispergiert werden. Durch die Zerstäubung entstehen feine Flüssigkeitströpfchen, wodurch sowohl die Menge an Waschflüssigkeit als auch die Auswaschzeit wesentlich gegenüber bekannten Verfahren verringert wird.

Es ist, nach Anspruch 5, vorteilhaft, einen Hefe-Filterkuchen von 10 bis 20 mm aufzubauen. Bei dieser Kuchendicke ist noch eine gute Filtrationsleistung von 300 bis 800 l/m²/h bei einem Filtrationsdruck von 3–5 bar vorhanden.

Das Durchblasen des Hefe-Filterkuchens nach dem Entleeren des Filterbehälters befreit die Hefezellen von der sie umgebenden Flüssigkeit. Gemäß Anspruch 6 ist es zweckmäßig, mit Luft oder einem anderen Gas den Filterkuchen 3–5 Minuten, vorzugsweise 3 Minuten, durchzublasen. Der Verbrauch an Druckluft ist relativ gering. Zu Beginn tritt fast keine Luft durch den Filterkuchen. Nach 2–3 Minuten beginnt der Filterkuchen zu springen, wodurch das Ende des Ausblasens angezeigt wird.

Diese kurze Trocknungszeit ist ausreichend, um in überraschender Weise, gemäß Anspruch 7, einen Filterkuchen von wenigstens 29 Gew.-% Hefe T.S. zu erreichen. Je nach Arbeitsweise konnten Hefe T.S. von 29 Gew.% bis 33 Gew.% erreicht werden. Diese hohen T.S.-Gehalte können auf Trommelfiltern nur durch Zugabe von Kochsalz erreicht werden.

Gemäss Anspruch 8 kann der gewünschte Gehalt an Hefetrockensubstanz durch den Fitrationsdruck und/oder den Druck der Waschflüssigkeit geregelt werden.

Gemäß Anspruch 9 ist es zweckmäßig, die Filtration und das Auswaschen in Zyklen von 10–40 Minuten durchzuführen. Dabei kann das Totvolumen von Fermentationsbrühe und Waschwasser in einen speziellen Tank oder aber in ein oder mehrere zugeschaltete Filter gleicher Art erfolgen.

Es hat sich als vorteilhaft erwiesen, gemäß Anspruch 10, den Hefe-Filterkuchen mittels vorgewärmter Luft durchzublasen. Dadurch kann die Durchblaszeit weiter verringert werden.

Bei der Gewinnung von Hefe aus der alkoholischen Fermentation wie sie beispielsweise bei der Herstellung von Bier und Wein anfällt, ist es gemäß Anspruch 11 zweckmäßig, den Filterbehälter sowohl auf der Trübseite als auch auf der Klarseite direkt mit dem Gärtank zu verbinden und so die ganze Filtration unter sterilen Bedingungen durchzuführen.

Das Verfahren hat sich, gemäß Anspruch 12, besonders zur Filtration von Bäckereihefe und zur Abtrennung von Hefe aus Bierherstellung und Weinbereitung bewährt.

Das Verfahren soll anhand einer Zeichnung beispielhaft näher beschrieben werden.

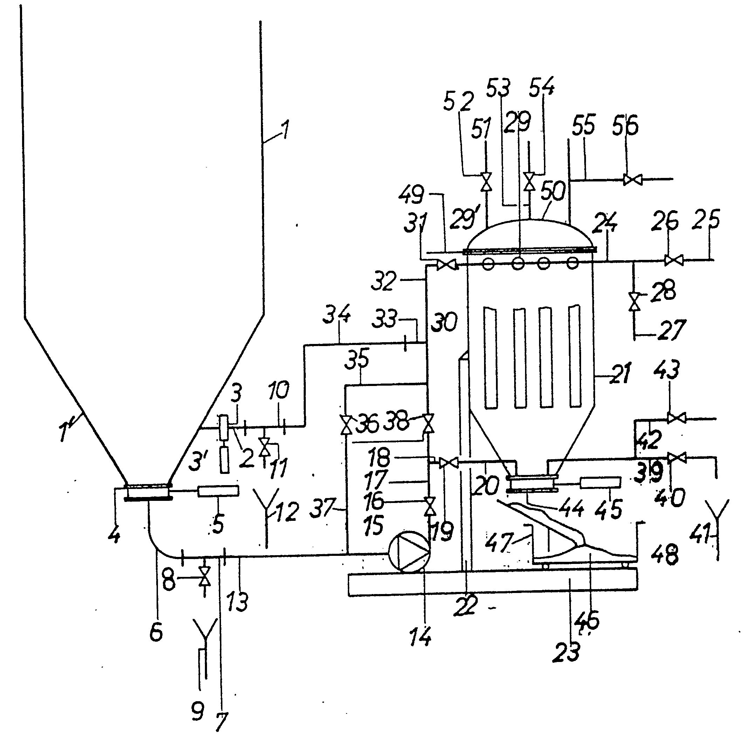

Gemäß der einzigen Figur wird ein Gärtank mit 1 bezeichnet. Der Gärtank 1 besteht aus einem zylindrischen oberen Teil und einem konischen unteren Teil 1'. Im konischen Teil 1' ist ein Stutzen 2 mit einem Ventil 3 mit einem Motor 3' angebracht. Im Apex des Gärtanks 1 ist ein Abschlußventil 4 vorgesehen, welches durch einen Stellmotor 5 angetrieben wird. Das Abschlußventil 4 ist über eine Leitung 6 mit einem Rohr 7 verbunden, an welchem ein Ventil 8 in einen Abfluß 9 mündet. Auch der Stutzen 2 ist mit einem Rohrteil 10 verbunden, welcher ein Ventil 11 trägt, das in einen Abfluß 12 mündet. Eine Schlauchverbindung 13 führt auf eine Pumpe 14. Von der Pumpe 14 führt eine Druckleitung 15 über ein Ventil 16 auf ein Leitungsstück 17, eine Abzweigleitung 18 über ein Ventil 19 und eine Zufuhrleitung 20 in den konischen Teil eines Druck-Filterbehälters 21 eines Kerzenfilters. Der Filterbehälter 21 ist auf einem Gestell 22 und einer Bodenplatte 23 befestigt. Der Behälter des Kerzenfilters ist mit der Zufuhrleitung 20 einer Spülleitung 24 mit einem Anschluß 25 für Waschwasser und einem Ventil 26 sowie einem Anschluß 27 mit einem Ventil 28 für Luft oder ein anderes Gas versehen. Die Leitung 24 führt durch den Filterbehälter 21 hindurch über Ventile 29, 29'. Im Inneren des Filterbehälters 21 sind Kerzenfilterelemente 30 reihenförmig hintereinander auf je einem Sammelrohr angeordnet. Jedes gezeichnete Filterelement 30 stellt ein Segment dar. Die Anzahl der Filterelemente 30 wird lediglich durch die Behältergröße begrenzt. Im Ausgangsteil der Leitung 24 ist ein Ventil 31 installiert. Es führt auf eine Leitung 32, welche einen Anschluß 33 für einen Schlauch 34 aufweist. Die Leitung 32 weist einen weiteren Abzweig 35 auf, welcher über ein Ventil 36 auf eine Leitung 37 führt, die mit der Pumpe 14 in Verbindung steht. Die Leitung 32 führt über ein Ventil 38 auf die Leitung 17. Im unteren Teil des Filterbehälters 21 ist eine weitere Leitung 39 vorgesehen, welche über ein Ventil 40 in einen Kondensatablauf 41 führt. Eine Bypass-Leitung 42 ist über ein Ventil 43 mit einer nicht gezeigten Druckgasquelle verbunden. Ein Schieber 44 mit einem Motor 45 dient zum Austragen der festen Rückstände 46 über eine Fördereinrichtung 47 in eine Transportvorrichtung 48. Der Filterbehälter 21 wird durch eine Platte 49, auf welcher die Kerzenfilterelemente 30 hängend befestigt sind, in einen unteren Trübraum und einen oberhalb der Filterelemente 30 befindlichen Filtratraum unterteilt. Im Deckel 50 des Behälters sind eine Anschlußleitung 51 mit einem Ventil 52 für Dampf, eine Anschlußleitung 53 mit einem Ventil 54 zur Entlüftung und eine Anschlußleitung 55 mit einem Ventil 56 für ein Druckgas vorgesehen.

Beispiel 1

Bei der Herstellung von Bier erfolgt nach der Ankuppelung des Filters an den Gärtank 1 zunächst die Sterilisation des Filterbehälters 21. Dazu wird Dampf von ca. 2 bar über das geöffnete Ventil 52 und die Anschlußleitung 51 bei den geöffneten Ventilen 40, 29, 29', 19, 16, 38, 36, 31, 11 und 8 aufgegeben. Es wird während 15 Minuten gedämpft. Nach der Abkühlung des Filters wird das Anschlußventil 4 partiell geöffnet. Die Hefesuspension fließt über die Leitung 6, das Rohr 7 und den Schlauch 13 zur Pumpe 14. Sie wird über die Leitung 15, das Ventil 16, das Leitungsstück 17, die Abzweigleitung 18, das geöffnete Ventil 19 und die Zufuhrleitung 20 in den Filterbehälter 21 gepumpt. Auf den Geweben der Kerzenfilterelemente 30 scheidet sich die Hefe ab. Das Filtrat fließt über die Ventile 29, 29', die Leitung 24 und das Ventil 31 über die Leitung 32, den Schlauch 34, das Rohrteil 10 und das geöffnete Ventil 3 zurück in den Gärtank 1. Nachdem der Filterkuchen auf den Kerzenfilterelementen 30 die vorbestimmte Dicke von beispielsweise 10–20 mm erreicht hat, wird die Filtration unterbrochen und das Restvolumen beispielsweise mittels Druckluft über die Leitung 55 bei geöffneten Ventilen 19, 38, 36 und 4 in den Gärtank 1 zurückgedrückt. Danach erfolgt das Ausblasen des Hefe-Filterkuchens zur Entwässerung. Hierzu wird Druckluft über das geöffnete Ventil 43 und die Leitung 42 auf den Behälter in Filrationsrichtung bei geöffnetem Entlüftungsventil 54 aufgegeben. Zur Entladung des Filterkuchens wird der Schieber 44 mittels des Elektromotors 45 geöffnet und Druckluft entgegengesetzt zur Filtrationsrichtung bei zeitlich hintereinander geöffneten Ventilen 29, 29' stoßweise aufgegeben. Der Filterkuchen fällt in Form von Hefe 46 mit einem Trockensubstanzgehalt von 29–33 Gew.-%, vorzugsweise 29–30 Gew.-%, über die Fördereinrichtung 47 in den Transportbehälter 48. Danach wird der Kerzenfilter über die Leitung 25, bei geöffneten Ventilen 26, 54 und 42 gewaschen und über den Schieber 44 entleert.

Mit der beschriebenen Vorrichtung ist es möglich, in einem Filtrationszyklus die im Gärtank 1 anfallende Hefemenge zu filtrieren.

Beispiel 2

Im Betrieb erfolgt die Filtration von Bäckereihefe aus dem Gärtank 1 wie folgt.

Eine bestimmte Menge einer Hefesuspension wird kontinuierlich über das eingestellte Ventil 4, die Leitung 6, das Rohr 7, den Schlauch 13 über die Pumpe 14, die Leitung 15, das Ventil 16, die Leitungsstücke 17 und 18, das Ventil 19 und die Leitung 20 in das Kerzenfilter gepumpt. Die Hefesuspension hat eine Konzentration von 3–5% Hefetrockensubstanz (HTS). Nachdem der Filterbehälter 21 gefüllt ist, scheidet sich die Hefe auf den Geweben der Kerzenfilterelemente 30 ab. Das Filtrat, welches praktisch hefefrei ist, kann über die Leitung 24 und das Ventil 26 abgeführt werden.

Wenn in den ersten Sekunden oder Minuten das Filtrat nicht genügend klar ist, wird es bei geöffnetem Ventil 31 und den geschlossenen Ventilen 26, 28, 36 und 38 in den Gärtank 1 zurückgeführt.

Nach etwa 10 Minuten wird bei einem Zulaufdruck von etwa 5 bar filtriert, wobei sich in dieser Zeit ein Hefekuchen von 10 bis 20 mm, vorzugsweise von 10–12 mm Dicke auf dem Gewebe der Filterkerzenelemente 30 aufgebaut hat. Nun wird die Pumpe 14 abgestellt und Druckluft von ca. 2 bar über die Anschlußleitung 55 bei geöffnetem Ventil 56 aufgegeben. Die restliche, im Filterbehälter 21 vorhandene Hefesuspension wird durch den Druck entweder über die Leitung 20, das geöffnete Ventil 19, die Leitung 18, das Ventil 38, den Schlauch 34 und das Ventil 3 in den Gärtank 1 zurückgedrückt oder über die Leitungen 39, 42 und das Ventil 43 in einen nicht gezeigten zweiten Filter gleicher Art filtriert. Nach der Entleerung des Restvolumens wird immer noch ein positiver Druck aufrecht erhalten, damit der Filterkuchen fest auf die Oberfläche der Filtergewebe gedrückt wird. Es kann nun Waschwasser über die Pumpe 14 in den Filterbehälter während ca. 5–8 Minuten gepumpt und so der Filterkuchen gewaschen werden. Das zuerst braune, verschmutzte Waschwasser verläßt den Filterbehälter 21 über die Leitungen 24 und 25 bei geöffnetem Ventil 26.

Nach beendeter Waschung des Filterkuchens wird das im Behälter vorhandene Restwasser über die Leitung 39 mit Druckluft aus der Leitung 55 entleert. Nach Entfernung des Waschwassers wird die Druckluft auf über 5 bar über die Leitungen 42 und 39 erhöht und der Filterkuchen durchgeblasen. Bereits nach 1–2 Minuten, spätestens nach 3–5 Minuten, beginnt der Hefefilterkuchen Sprünge aufzuweisen. Nun ist die Trocknung praktisch beendet. Der Luftverbrauch ist außerordentlich gering, da der Filterkuchen bis zur Rißbildung sehr schwer luftdurchlässig ist. Der Hefe-Filterkuchen weist einen Trockengehalt von 29 bis 33 Gew.-% auf, abhängig vom angewandten Gasdruck.

Nach beendeter Entwässerung wird der Schieber 44 geöffnet und der Hefefilterkuchen im Gegenstrom mittels Luft abgeworfen. Die gewonnene Hefe ist bereit für die Verpackung und für eine eventuelle weitere Trocknung zu Trockenbackhefe.

Bezeichnungen
1 Gärtank
1' konischer Teil des Gärtanks
2 Stutzen
3 Ventil
4 Abschlußventil
5 Stellmotor
6 Leitung
7 Rohr
8 Ventil
9 Abfluß
10 Rohrteil
11 Ventil
12 Abfluß
13 Schlauchverbindung
14 Pumpe
15 Druckleitung
16 Ventil
17 Leitungsstück
18 Abzweigleitung
19 Ventil
20 Zufuhrleitung
21 Filterbehälter
22 Gestell
23 Bodenplatte
24 Spülleitung
25 Anschluß
26 Ventil
27 Anschluß
28 Ventil
29 Ventil
29' Ventil
30 Kerzenfilterelemente
31 Ventil
32 Leitung
33 Anschluß
34 Schlauch
35 Abzweig
36 Ventil
37 Leitung
38 Ventil
39 Leitung
40 Ventil
41 Kondensatablauf
42 Bypass-Leitung
43 Ventil
44 Schieber
45 Motor
46 Hefe
47 Fördereinrichtung
48 Transporteinrichtung
49 Platte
50 Deckel
51 Anschlußleitung
52 Ventil
53 Anschlußleitung
54 Ventil
55 Anschlußleitung
56 Ventil

**Patentansprüche**

1. Verfahren zur Abtrennung von Hefen aus den Fermentationsbrühen eines Gärtanks (1), insbesondere zur Herstellung von gepreßter Hefe für Backzwecke sowie gepreßter Hefe aus der alkoholischen Vergärung die bei der Alkohol-, Wein- und Bierbereitung anfällt, dadurch gekennzeichnet, daß die Fermentationsbrühe durch einen Druckfilter mit frei in einem Filterbehälter (21) aufgehängten Filterelementen (30) gepreßt wird, dessen Filterelemente (30) mit feinem Filtergewebe oder Membranen überzogen sind, der Filterbehälter (21) nach Aufbau eines Filterkuchens von der Restflüssigkeit entleert, der Hefe-Filterkuchen mit Wasser gewaschen, mit Druckluft oder einem anderen Druckgas ausgeblasen und durch einen Druckstoß mit Druckgas entgegengesetzt zur Filtrationsrichtung von den Filtergeweben gelöst und aus dem Filterbehälter (21) ausgetragen wird.

2. Verfahren nach Anspruch 1, dadurch gekennzeichnet, daß der Filterbehälter (21) direkt mit dem Gärtank (1) verbunden ist.

3. Verfahren nach den Ansprüchen 1 und 2, dadurch gekennzeichnet, daß während des Wasch- und Ausblasvorgangs, sowie während des Auftragens des Hefe-Filterkuchens im Behälter des Druckfilters (21) ein Überdruck herrscht.

4. Verfahren nach den Ansprüchen 1 bis 3, dadurch gekennzeichnet, daß zum Auswaschen des Hefe-Filterkuchens die Waschflüssigkeit in Luft oder einem anderen Gas dispergiert wird.

5. Verfahren nach den Ansprüchen 1 bis 4, dadurch gekennzeichnet, daß der Hefe-Filterkuchen eine Dicke von 10 bis 20 mm aufweist.

6. Verfahren nach den Ansprüchen 1 bis 5, dadurch gekennzeichnet, daß das Ausblasen des Hefe-Filterkuchens mit Luft oder einem anderen Gas in 3–5 Minuten erfolgt.

7. Verfahren nach den Ansprüche 1 bis 6, dadurch gekennzeichnet, daß der Hefe-Filterkuchen wenigstens 29 Gew.-% Hefe-Trockensubstanz enthält.

8. Verfahren nach Anspruch 7, dadurch gekennzeichnet, daß der Trockengehalt des Hefe-Filterkuchens durch den Filtrations- und/oder den Druck der Waschflüssigkeit geregelt wird.

9. Verfahren nach den Ansprüchen 1 bis 8, dadurch gekennzeichnet, daß die Filtration und das Auswaschen in Zyklen von 10–40 Minuten erfolgt.

10. Verfahren nach Anspruch 4, dadurch gekennzeichnet, daß das Durchblasen des Hefe-Filterkuchens mit vorgewärmter Luft erfolgt.

11. Verfahren nach den Ansprüchen 1 bis 10, dadurch gekennzeichnet, daß bei der Gewinnung von Hefe aus der alkoholischen Fermentation der Filterbehälter (21) auf der Trübseite und auf der Klarlaufseite direkt mit dem Gärtank (1) verbunden ist.

12. Anwendung des Verfahrens nach den Ansprüchen 1 bis 11 zur Filtration von Bäckereihefen und zur Abtrennung von Hefen bei der Bierherstellung und Weinbereitung.

## Claims

1. A process for separation of yeasts from fermentation broths of a fermentation tank (1) particularly for the production of compressed yeast for baking purposes, as well as compressed yeast from alcoholic fermentation which accrues in the production of alcohol, wine and beer, characterised in that the fermentation broth is forced through a pressure filter with filter elements (30) freely suspended within a filter vessel (21) the filter elements (30) of which are covered with fine filter fabrics or membranes, that after the build-up of a filter cake, the filter vessel (21) is drained of the residual liquid, the yeast filter cake is washed with water, blown out with compressed air or another compressed gas and freed from the filter fabrics by means of a pressure surge with compressed gas in the opposite direction to that of filtration and extracted from the filter vessel (21).

2. A process according to claim 1, characterised in that the filter vessel (21) is connected directly to the fermentation tank (1).

3. A process according to claims 1 and 2, characterised in that an overpressure prevails during the washing and blowing-out operations, as well as during the accretion of the yeast filter cake within the vessel of the pressure filter (21).

4. A process according to claims 1 to 3, characterised in that for washing out the yeast filter cake, the washing liquid is dispersed in air or in another gas.

5. A process according to claims 1 to 4, characterised in that the yeast filter cake has a thickness of 10 to 20 mm.

6. A process according to claims 1 to 5, characterised in that the blowing out of the yeast filter cake by means of air or of another gas is carried out in 3 to 5 minutes.

7. A process according to claims 1 to 6, characterised in that the yeast filter cake contains at least 29% by weight of dry yeast substance.

8. A process according to claim 7, characterised in that the dry content of the yeast filter cake is controlled by means of the filtration pressure and/or of the pressure of the washing liquid.

9. A process according to claims 1 to 8, characterised in that the filtering and washing-out occur in cycles of 10 to 40 minutes.

10. A process according to claim 4, characterised in that the blowing-through of the yeast filter cake is carried out with preheated air.

11. A process according to claims 1 to 10, characterised in that during the production of yeast from alcoholic fermentation, the filter vessel (21) is directly connected to the fermentation tank (1) on the turbid side and on the clear filtrate side.

12. Application of the process according to claims 1 to 11 for the filtering of bakery yeasts and for separation of yeasts in beer production and wine making.

## Revendications

1. Procédé de séparation de levure de bouillons de culture d'un réservoir de fermentation (1), en particulier pour la fabrication de levure comprimée pour la cuisson, ainsi que de levure comprimée provenant de la fermentation alcoolique qui a lieu lors de la préparation d'alcool, de vin et de bière, caractérisé par le fait qu'on comprime le bouillon de culture à travers un filtre à pression comportant des éléments filtrants (30) suspendus librement dans un récipient de filtration (21), lesdits éléments filtrants (30) étant recouverts d'un tissu filtrant fin ou de membranes, qu'après la formation d'un gâteau de filtration, on vide le récipient filtrant (21) du liquide restant, qu'on lave à l'eau le gâteau de filtration de levure, qu'on insuffle de l'air comprimé ou un autre gaz comprimé à travers le gâteau, qu'on le détache des tissus filtrants par une impulsion de pression avec du gaz comprimé, opposée au sens de filtration, et qu'on l'évacue hors du récipient de filtration (21).

2. Procédé selon la revendication 1, caractérisé par le fait que le récipient de filtration (21) est relié directement au réservoir de fermentation (1).

3. Procédé selon les revendications 1 et 2, caractérisé par le fait qu'il règne une surpression dans le récipient du filtre à pression (21), pendant le déroulement du lavage et de l'insufflation, de même que pendant la formation du gâteau de filtration de levure.

4. Procédé selon les revendications 1 à 3, caractérisé par le fait que, pour le lavage du gâteau de filtration de levure, on disperse le liquide de lavage dans l'air ou un autre gaz.

5. Procédé selon les revendications 1 à 4, caractérisé par le fait que le gâteau de filtration de levure présente une épaisseur de 10 à 20 mm.

6. Procédé selon les revendications 1 à 5, caractérisé par le fait qu'on effectue l'insufflation à travers le gâteau de filtration de levure avec de l'air ou un autre gaz, en l'espace de 3–5 minutes.

7. Procédé selon les revendications 1 à 6, caractérisé par le fait que le gâteau de filtration de levure contient au moins 29% en poids de matière sèche de levure.

8. Procédé selon la revendication 7, caractérisé par le fait qu'on règle la teneur en matière sèche du gâteau de filtration de levure par la pression de filtration et/ou la pression du liquide de lavage.

9. Procédé selon les revendications 1 à 8, caractérisé par le fait que la filtration et le lavage ont lieu dans des cycles de 10–40 minutes.

10. Procédé selon la revendication 4, caractérisé par le fait qu'on effectue l'insufflation à travers le gâteau de filtration de levure avec de l'air préchauffé.

11. Procédé selon les revendications 1 à 10, caractérisé par le fait que, dans la production de levure par la fermentation alcoolique, on relie le récipient de filtration (21) directement au réservoir de fermentation (1), du côté de la suspension et du côté de l'écoulement du liquide clair.

12. Application du procédé tel que défini aux revendications 1 à 11 pour la filtration de levures de boulangerie et pour la séparation de levures dans la fabrication de bière et dans la préparation du vin.